# EUROPEAN PATENT SPECIFICATION

(11) **EP 0 961 620 B1**
(45) Date of publication and mention of the grant of the patent: **13.02.2002**
(21) Application number: 97912579.6
(22) Date of filing: 03.11.1997
(51) Int. Cl.: A61L 2/16, A61L 9/00

(54) **METHOD FOR TREATMENT OF WET ORGANIC WASTE**
VERFAHREN ZUR BEHANDLUNG VON FEUCHTEM ORGANISCHEN ABFALL
PROCEDE DE TRAITEMENT DE DECHETS ORGANIQUES HUMIDES

(30) Priority: 08.11.1996 NO 964762
(43) Date of publication of application: 08.12.1999
(73) Proprietor: NORSK HYDRO A/S, 0240 Oslo 2 (NO)
(72) Inventor: HOYVIK, Henrik, N-3731 Skien (NO); HJ RNEVIK, Leif, N-3714 Skien (NO); GRANLI, Tom, N-3173 Vear (NO); OLSEN, Britt, Gunhild, N-3924 Porsgrunn (NO)
(74) Representative: Bleukx, Lucas Lodewijk M.
(86) International application number: NO9700289
(87) International publication number: WO9820911

(56) References cited:
- WO-A-95/16470
- DE-A- 3 329 338
- DE-A- 3 810 281
- DE-A- 3 903 825
- GB-A- 1 452 768
- NL-A- 9 300 611
- US-A- 5 568 895

## Description

The present invention relates to treatment of household biowaste collected in containers, for reduction of odour and microbial activity, comprising adding a treatment agent at least once during a storage period before the treated biowaste is further processed or deposited. The invention also comprises use of a special type of treatment agent.

Biowaste comprises household waste collected in 150-200 litre bins for separate collection of wet organic waste fractions. Such waste can be placed in the bins several times a week and the bins are usually emptied after 7-14 days and transported to landfills or composting plants. Problems may occur during collection and storage of biowaste such as odour, flies and larvae, especially during the summer. Odour is also one of the main problems during composting. Accordingly, there is a need for treatment of biowaste to reduce and prevent odour and microbial activity. However, the biowaste should not be treated in such a way that problems arise during further processing or deposition of said waste. Special problems may arise if the treated biowaste shall be further processed to feedstuff. Then the treatment agent should preferably be completely degraded, and the products thereof must be acceptable in the feedstuff. Remaining treatment agent should also be acceptable.

Treatment of biowaste with nitrates like calcium nitrate, will under anaerobic condition reduce and/or prevent formation of hydrogen sulphide. Odour due to presence of hydrogen sulphide, organic acids, organic nitrogen and sulphur compounds can be substantially reduced by application of nitrates, but the positive effects thereof are limited to anaerobic conditions. The effect of nitrate addition on various microbial activities resulting in odour formation, larvae etc. is also very limited.

Treatment of biowaste with organic acids like formic acid is known in the art. The effect on microbial activity has proved to be positive. The corrosion and etching problems related to such a treatment agent are, however, strongly limiting its use. In the case of further processing the biowaste it is essential that there has not been applied too much acid as remains will cause various unacceptable problems.

From NL-A-9300611 there is known a process for counteracting the problems caused by decomposition of organic waste, in particular vegetable, fruit and garden waste in biocontainers, comprising treating the waste with a solution of one or several organic acids in an amount of 0.05-5 weight% of the solution with regard to the amount of waste. The organic acid diluted with water may comprise ammonium or (earth) alkali ions, for instance ammonium diformate. Preferably an odour improving agent such as citric acid is added to the treatment solution.

The main object of the invention was to arrive at a method of treating biowaste that would allow the waste to be stored for about 3 weeks without getting problems with odours, flies, larvae etc. and prevent/reduce microbial growth. Said treatment should not result in problems during further processing or depositing of the treated biowaste.

Another object was that the treated biowaste should be useful for further processing to compost or feedstuff.

A further object was that the treatment agent used should not cause any harm to the people being exposed to said agent during handling and processing of the biowaste.

The inventors started the development of a new method for treatment of biowaste by evaluating the various criteria to be met with regard to the treatment process itself and the resulting product, especially with regard to its further processing to foodstuff and compost.

Firstly, the treatment should result in substantial reduction in odour from the biowaste during storage for up to 3 weeks. Microbial activity and growth should be stopped or at least be kept at a very low level. Employed treatment agent should be biodegradable and remains thereof or its possible reaction products should not cause any problems in further processing of treated biowaste. In order to reduce/stop microbial activity, the pH of the biowaste/treatment agent mixture should preferably be below pH 4. Said mixture and the treatment agent should also be within acceptable ranges with regard to etching and corrosion to equipment and those exposed thereto during handling and processing.

When the treated biowaste is further processed to compost, the ideal pH would be in the range of pH 5-9 during the composting process, which initially also should have a minimum of microbial activity. If the treated biowaste shall be further processed to feedstuff, there are some general requirements to be met. Said biowaste shall not contain visible amounts of mould fungi, rod bacilli, larvae etc. indicating bacterial activity.

In view of the above, partly conflicting criteria and requirements, the inventors investigated various possible useful treatment agents. Salts of formic acid and similar acids were investigated, and it was then found that especially some of the disalts of such acids, possibly admixed with the corresponding acid, seemed to be applicable. Initially tests were performed to qualitatively determine the effects with regard to odour. These tests showed promising results provided sufficient amounts of treatment agent were evenly distributed throughout the biowaste. Even distribution could best be obtained if the biowaste had a minimum moisture content and the treatment agent was spread on the biowaste in the form of liquid/aqueous solutions.

Though formates comprising formic acid proved to give best effect, acetate or propionate comprising the corresponding acids were also found to be applicable.

The initial tests were followed up with similar treatment of the biowaste, but then recording and analysing the treated biowaste with regard to microbial activity, mould fungi and rod bacilli. Presence of larva in the biowaste during storage and at the end of the treatment periods for the biowaste were also recorded. During these experiments it was then found that the treatment with the various formate solutions could prevent and substantially reduce microbial activity for a prolonged time. Series of tests were then performed to verify the initial promising results. Further, the treated biowaste was further processed by composting and it was then found that this would be possible provided necessary amounts of bacteria were present for starting the composting process.

The further experiments showed that biowaste treated according to the invention as described in claim 1 with an aqueous solution of formate, acetate, or propionate, comprising their respective acids and disalts thereof, by spraying said solution in amounts of 1-3 weight%, calculated as formate/acetate/propionate equivalents, on the biowaste having a moisture content of 50-75 weight%, would have the odour and microbial activity substantially reduced.

Sodium-, ammonium or potassium diformate, especially the latter, proved to be especially effective in treating household waste. Preferred treatment would be a 15-50 weight% solution of potassium diformate sprayed on the biowaste each time it was supplied to the collecting bin in amounts of 50-70 ml/kg wet biowaste.

An aqueous solution of formic acid and formate in a ratio of 1:1 to 1:2 would also be a useful treatment agent.

Composting of biowaste treated according to the invention could be performed by mixing the biowaste with bark, turf or the like in amounts of 25-75 weight% of the total mixture which then was subjected to aeration. Before or during the initial composting process the biowaste/bark mixture could be inoculated with minor amounts of adapted micro-organisms, for instance by adding untreated biowaste or compost.

The scope and special features of the invention are as defined in the attached claims.

The invention is further explained in connection with the following examples.

### Example 1

This example shows the effects of treatment of wet household waste with various treatment agents with regard to microbial growth. The effects on rod bacilli in household waste as function of treatment of different treatment agents are shown.

The experiments were carried out by filling household waste in 9 buckets, one used for control. On day one, 2.5 kg household waste was filled in each bucket and a treatment solution was sprayed on said waste. Later on 0.5 kg waste was filled in the buckets on each waste and treatment addition to the buckets. The experiment lasted for 22 days. The amount of mould fungi or rod bacilli were measured as colony forming units per gram (Cfu/g), as a function of time, type and amount of treatment agent. In the Tables 2-13, E means exponential. Samples for analysis were taken from the top (T) and bottom (B) layers of waste in the buckets. Each sample contained 50 grams household waste.

Table 1 shows type of treatment agent, amount applied, expressed as % formate equivalent, and sampling distribution. In the following tables the diformates are expressed as (50/50) or (70/30), which means that the treatment agent comprises an aqueous solution of 60 weight% diformate and 40 weight% water or a mixture of 1000 grams of this solution with 300 grams of 94% formic acid. The Controls, C-1 for the tests with potassium diformate and C-2 for the control in the comparative experiments, refer to addition of water only to the buckets each time household waste is filled therein, In the comparative experiments the effects of addition of formates are compared with addition of a mixture of Ca-, NH₄-, Na- and K-nitrates.

**Table 1**

| Bucket | Agent | % Form. | Day T | 5 B | Day T | 10 B | Day T | 17 B | Day T | 22 B |
|---|---|---|---|---|---|---|---|---|---|---|
| A | K-diformate (50/50)¹ | 1.5 | 2 | 1 | 2 | 1 | 2 | 1 | 2 | 1 |
| B | K-diformate (70/30) | 1.5 | 2 | 1 | 2 | 1 | 2 | 1 | 2 | 1 |
| C | K-diformate (50/50) | 2.5 | 2 | 1 | 2 | 1 | 2 | 1 | 2 | 1 |
| D | K-diformate (70/30) | 2.5 | 2 | 1 | 2 | 1 | 2 | 1 | 2 | 1 |
| E | NH₃-diformate (50/50) | 2.5 | 2 | 1 | 2 | 1 | 2 | 1 | 2 | 1 |
| F | NH₃-diformate (70/30) | 2.5 | 2 | 1 | 2 | 1 | 2 | 1 | 2 | 1 |
| G | Nitrate | | 2 | 1 | 2 | 1 | 2 | 1 | 2 | 1 |
| H | Nitrate | | 2 | 1 | 2 | 1 | 2 | 1 | 2 | 1 |
| C-1 | Water | | 2 | 1 | 2 | 1 | 2 | 1 | 2 | 1 |
| C-2 | Water | | 2 | 1 | 2 | 1 | 2 | 1 | 2 | 1 |

The results from the aerobic gram negative rod bacilli in the samples from the top layer are shown in Table 2.

**Table 2**

| Bucket | Agent | Day 1 | Day 5 | Day 10 | Day 17 | Day 22 |
|---|---|---|---|---|---|---|
| A | K-diform(50/50) 1.5% | 6.00E +06 | 2.50E +05 | 9.75E +06 | 3.18E +07 | 2.80E +04 |
| B | K-diform(70/30) 1.5% | 6.00E +06 | 1.75E +05 | 1.00E +00 | 2.75E +07 | 4.25E +03 |
| C | K-diform(50/50) 2.5% | 6.00E +06 | 1.00E +00 | 7.50E +05 | 1.00E +00 | 4.75E +03 |
| D | K-diform(70/30) 2.5% | 6.00E +06 | 1.00E +00 | 1.00E +00 | 5.00E +03 | 1.00E +00 |
| C-1 | Water | 6.00E +06 | 1.25E +08 | 2.13E +09 | 1.75E +10 | 4.31E +10 |

The results from the bottom layer for comparative tests are shown in Table 3.

**Table 3**

| Bucket | Agent | Day 1 | Day 5 | Day 10 | Day 17 | Day 22 |
|---|---|---|---|---|---|---|
| E | NH₃diform(50/50) 2.5% | 6.00E+06 | 2.00E+05 | 1.38E+06 | 5.00E+02 | 1.00E+00 |
| F | NH₃diform(70/30) 2.5% | 6.00E+06 | 7.50E+02 | 1.25E+06 | 1.00E+00 | 1.00E+00 |
| G | Nitrate | 6.00E+06 | 2.25E+07 | 2.50E+07 | 7.83E+08 | 9.50E+07 |
| H | Nitrate | 6.00E+06 | 1.01E+07 | 6.25E+07 | 7.75E+07 | 9.75E+07 |
| C-2 | Water | 6.00E+06 | 7.50E+06 | 2.13E+09 | 1.75E+10 | 4.31E+10 |

The results from the bottom layer for agents being potassium diformate are shown in Table 4.

**Table 4**

| Bucket | Agent | Day 1 | Day 5 | Day10 | Day 17 | Day 22 |
|---|---|---|---|---|---|---|
| A | K-diform(50/50) 1.5% | 6.00E+06 | 5.00E+05 | 2.70E+04 | 3.50E+03 | 2.00E+03 |
| B | K-diform(70/30) 1.5% | 6.00E+06 | 1.00E+00 | 1.30E+06 | 6.00E+06 | 7.00E+06 |
| C | K-diform(50/50) 2.5% | 6.00E+06 | 1.00E+03 | 2.00E+03 | 2.50E+03 | 3.00E+03 |
| D | K-Diform(70/30) 2.5% | 6.00E+06 | 1.00E+00 | 1.00E+00 | 1.00E+00 | 1.00E+00 |
| C-1 | Water | 6.00E+06 | 1.00E+07 | 4.75E+07 | 1.48E+06 | 3.25E+05 |

The results from the comparative experiments for the bottom layer are shown in Table 5.

**Table 5**

| Bucket | Agent | Day 1 | Day 5 | Day 10 | Day 17 | Day 22 |
|---|---|---|---|---|---|---|
| E | NH₃-diform(50/50) 2.5% | 6.00E+06 | 1.00E+00 | 3.00E+03 | 2.40E+02 | 2.00E+03 |
| F | NH₃-diform(70/30) 2.5% | 6.00E+06 | 1.00E+00 | 1.00E+07 | 2.50E+03 | 5.00E+02 |
| G | Nitrate | 6.00E+06 | 1.00E+00 | 6.00E+07 | 1.00E+07 | 4.74E+04 |
| H | Nitrate | 6.00E+06 | 5.00E+06 | 4.50E+07 | 2.40E+06 | 9.00E+05 |
| C-2 | Water | 6.00E+06 | 1.00E+07 | 4.75E+07 | 1.48E+06 | 3.25E+05 |

Experiments corresponding to those above for aerobic conditions were run under anaerobic conditions for negative rod bacilli. The results in the top layers for treatment with potassium diformate are shown in Table 6.

**Table 6**

| Bucket | Agent | Day 1 | Day 5 | Day 10 | Day 17 | Day 22 |
|---|---|---|---|---|---|---|
| A | K-diform(50/50) 1.5% | 1.00E+00 | 1.00E+00 | 7.50E+03 | 1.76E+06 | 1.00E+00 |
| B | K-diform(70/30) 1.5% | 1.00E+00 | 1.00E+00 | 1.50E+05 | 3.35E+06 | 1.00E+00 |
| C | K-diform(50/50) 2.5% | 1.00E+00 | 1.00E+00 | 2.50E+04 | 1.00E+00 | 1.00E+00 |
| D | K-diform(70/30) 2.5& | 1.00E+00 | 1.00E+00 | 1.00E+00 | 7.50E+02 | 1.00E+00 |
| C-1 | Water | 1.00E+00 | 3.75E+02 | 1.25E+08 | 7.50E+07 | 1.48E+08 |

The results for the comparative agents in the top layer are shown in Table 7.

**Table 7**

| Bucket | Agent | Day 1 | Day 5 | Day 10 | Day 17 | Day 22 |
|---|---|---|---|---|---|---|
| E | NH₃-diform(50/50) 2.5% | 1.00E+00 | 1.00E+00 | 2.50E+04 | 1.00E+00 | 1.00E+00 |
| F | NH₃-diform(70/30) 2.5% | 1.00E+00 | 1.00E+00 | 4.25E+03 | 1.00E+00 | 1.00E+00 |
| G | Nitrate | 1.00E+00 | 2.50E+04 | 9.25E+05 | 7.00E+07 | 6.25E+04 |
| H | Nitrate | 1.00E+00 | 1.00E+00 | 2.50E+06 | 2.78E+06 | 1.60E+06 |
| C-2 | Water | 1.00E+00 | 3.75E+02 | 1.25E+08 | 7.50E+07 | 1.48E+08 |

The results for the bottom layer with treatment with potassium diformate are shown in Table 8.

**Table 8**

| Bucket | Agent | Day 1 | Day 5 | Day 10 | Day 17 | Day 22 |
|---|---|---|---|---|---|---|
| A | K-diform(50/50) 1.5% | 1.00E+00 | 1.00E+00 | 1.00E+00 | 1.00E+00 | 1.00E+00 |
| B | K-diform(70/30) 1.5% | 1.00E+00 | 1.00E+00 | 1.00E+00 | 1.00E+00 | 1.00E+00 |
| C | K-diform(50/50) 2.5% | 1.00E+00 | 1.00E+00 | 1.00E+00 | 1.00E+00 | 1.00E+00 |
| D | K-diform(70/30) 2.5% | 1.00E+00 | 1.00E+00 | 1.00E+00 | 1.00E+00 | 1.00E+00 |
| C-1 | Water | 1.00E+00 | 1.50E+05 | 3.25E+07 | 1.33E+04 | 3.25E+05 |

The results from the comparative tests in the bottom layer are shown in Table 9.

**Table 9**

| Bucket | Agent | Day 1 | Day 5 | Day 10 | Day 17 | Day 22 |
|---|---|---|---|---|---|---|
| E | NH₃-diform(50/50) 2.5% | 1.00E+00 | 1.00E+00 | 1.00E+00 | 1.00E+00 | 1.00E+00 |
| F | NH₃-diform(70/30) 2.5% | 1.00E+00 | 1.00E+00 | 1.00E+00 | 1.00E+00 | 1.00E+00 |
| G | Nitrate | 1.00E+00 | 1.00E+00 | 1.00E+06 | 1.00E+05 | 1.00E+00 |
| H | Nitrate | 1.00E+00 | 1.00E+00 | 1.00E+05 | 1.00E+03 | 1.00E+00 |
| C-2 | Water | 1.00E+00 | 1.50E+05 | 3.25E+07 | 1.33E+04 | 3.25E+05 |

From the results given in Tables 2-9 it can be seen that the presence of rod bacilli in the buckets treated with potassium diformate under aerobic conditions is lower than for the control buckets and the results are best with the highest amounts of treatment agent (buckets C and D). In the buckets which are treated with ammonium diformate there is a marked reduction, but with those treated with nitrate (buckets G and H) there is no marked difference compared with the control bucket C-2. The tendency is the same for the bottom layer as for the top layer samples.

The experiments performed at anaerobic conditions show a substantial reduction of the rod bacilli when the household waste is treated with potassium diformate and best results are obtained with the highest amounts of treatment agent. Treatment with nitrate gives hardly any effect for the top layer samples, and the same goes for the bottom layer samples. The effects of the treatment agent ammonium diformate is lower than for potassium diformate.

### Example 2

This example shows the results of tests performed as in Example 1 for recording the effects on mould fungi. The results of treatment with potassium diformate of the household waste on mould fungi growth are shown in Table 10.

**Table 10**

| Bucket | Agent | Day 1 | Day 5 | Day 10 | Day 17 | Day 22 |
|---|---|---|---|---|---|---|
| A | K-diform(50/50) 1.5% | 3.40E+03 | 5.00E+05 | 1.38E+06 | 9.00E+04 | 3.00E+03 |
| B | K-diform(70/30) 1.5% | 3.40E+03 | 2.85E+05 | 2.41E+06 | 1.15E+06 | 1.00E+00 |
| C | K-diform(50/50) 2.5% | 3.40E+03 | 6.50E+04 | 6.00E+05 | 1.00E+00 | 2.50E+02 |
| D | K-diform(70/30) 2.5% | 3.40E+03 | 8.00E+03 | 1.00E+00 | 1.08E+04 | 2.50E+02 |
| C-1 | Water | 3.40E+03 | 1.19E+07 | 3.80E+07 | 1.37E+07 | 9.44E+06 |

The results from the top layer for the comparative tests for mould fungi are shown in Table 11.

**Table 11**

| Bucket | Agent | Day 1 | Day 5 | Day 10 | Day 17 | Day 22 |
|---|---|---|---|---|---|---|
| E | NH₃-diform(50/50) 2.5% | 3.40E+03 | 1.63E+05 | 4.50E+05 | 2.10E+04 | 1.00E+00 |
| F | NH₃-diform(70/30) 2.5% | 3.40E+03 | 2.52E+05 | 3.00E+05 | 1.00E+00 | 1.00E+00 |
| G | Nitrate | 3.40E+03 | 6.45E+06 | 3.14E+06 | 1.13E+07 | 2.78E+07 |
| H | Nitrate | 3.40E+03 | 2.25E+06 | 6.30E+06 | 1.32E+06 | 2.13E+07 |
| C-2 | Water | 3.40E+03 | 1.19E+07 | 3.80E+07 | 1.37E+07 | 9.44E+06 |

The results on mould fungi growth from the bottom samples for household waste treated with potassium diformate are shown in Table 12.

**Table 12**

| Bucket | Agent | Day 1 | Day 5 | Day 10 | Day 17 | Day 22 |
|---|---|---|---|---|---|---|
| A | K-diform(50/50) 1.5% | 3.40E+03 | 3.50E+04 | 1.00E+00 | 1.00E+00 | 1.00E+00 |
| B | K-diform(70/30) 1.5% | 3.40E+03 | 1.00E+00 | 6.60E+04 | 7.00E+04 | 1.00E+00 |
| C | K-diform(50/50) 2.5% | 3.40E+03 | 1.00E+04 | 4.50E+03 | 1.00E+00 | 1.00E+00 |
| D | K-diform(70/30) 2.5% | 3.40E+03 | 1.00E+00 | 1.00E+00 | 1.00E+00 | 1.00E+00 |
| C-1 | Water | 3.40E+03 | 4.10E+06 | 2.40E+06 | 9.50E+05 | 8.50E+03 |

The results from the bottom layer for the corresponding comparative experiments are shown in Table 13.

**Table 13**

| Bucket | Agent | Day 1 | Day 5 | Day 10 | Day 17 | Day 22 |
|---|---|---|---|---|---|---|
| E | NH₃-diform(50/50) 2.5% | 3.40E+03 | 1.00E+00 | 1.00E+00 | 1.00E+00 | 1.00E+00 |
| F | NH₃-diform(70/30) 2.5% | 3.40E+03 | 1.00E+00 | 1.00E+00 | 1.00E+00 | 2.50E+03 |
| G | Nitrate | 3.40E+03 | 1.02E+07 | 4.00E+06 | 1.51E+05 | 1.50E+05 |
| H | Nitrate | 3.40E+03 | 7.55E+06 | 5.50E+06 | 1.00E+05 | 7.50E+04 |
| C-2 | Water | 3.40E+03 | 4.10E+06 | 2.40E+06 | 9.50E+05 | 8.50E+03 |

The results from the mould fungi experiments shown in Tables 10-13 clearly demonstrate that treatment of household waste with diformates, especially potassium diformate, substantially reduces the development of mould fungi. It is also evident that it is necessary to apply a minimum amount of treatment agent as shown by differences in effect when 1.5% and 2.5% treatment agent are used. The effect of ammonium diformate is somewhat lower than for potassium diformate. From the comparative tests it can be seen that treatment with nitrate hardly gives any effect compared with the control (C-2). The results are about the same for the top and bottom layers with regard to treatment with nitrate.

### Example 3

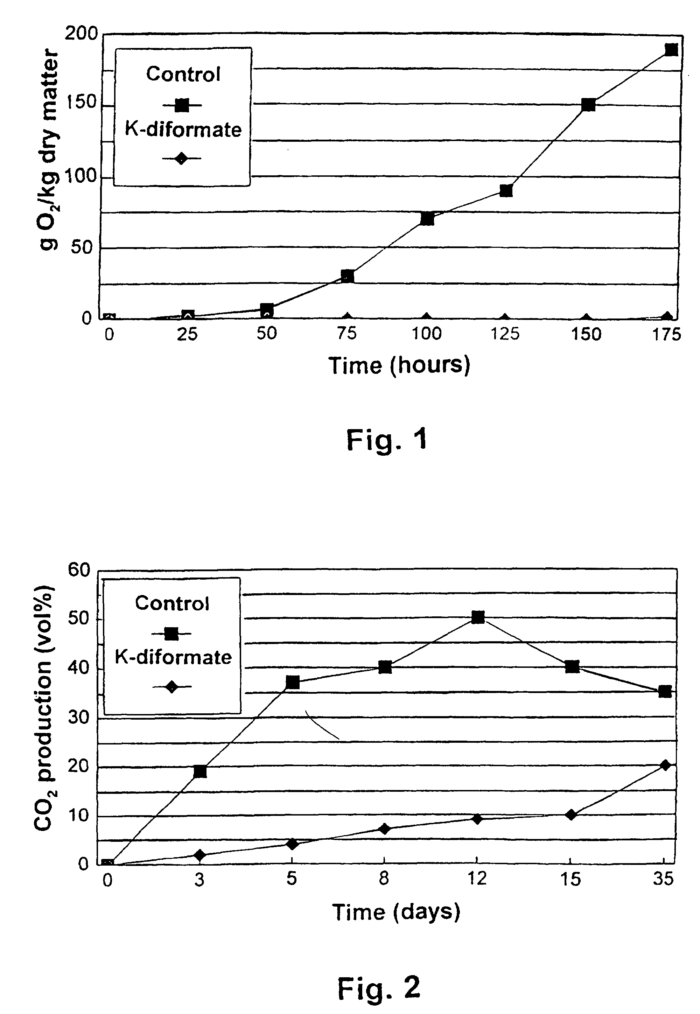

This example shows the effect of the treatment with a 2.5 weight% solution of potassium diformate under aerobic- and anaerobic condition on microbial degradation processes in wet household waste. The effect under aerobic conditions is shown as gram CO₂ formation/kg dry matter as function of time. The control curve of Figure 1 refers to addition of water only to the waste. The effect under anaerobic conditions is given as CO₂ production in volume% as function of time. This is shown in Figure 2, which clearly shows that the CO₂ production was stopped almost completely by the potassium diformate treatment, which means that the microbial activity was stopped correspondingly.

### Example 4

This example shows composting of household waste treated with and without a solution of potassium diformate in water. The dosage was 55 ml/kg wet waste. The initial water content for composting was adjusted to about 55%. The control experiments were performed by adding water instead of the diformate solution or inoculum. By inoculum is here meant compost which previously has been exposed to formate. The composting experiments were carried out in three 100 litre bioreactors, and a continuous aeration was achieved by means of compressed air. Bark was mixed with waste in a 1:1 weight ratio. The reactors were continuously aerated with 50 up to 100 l/h corresponding to an aeration of 1.7-1.75 l/kg dry matter per hour.

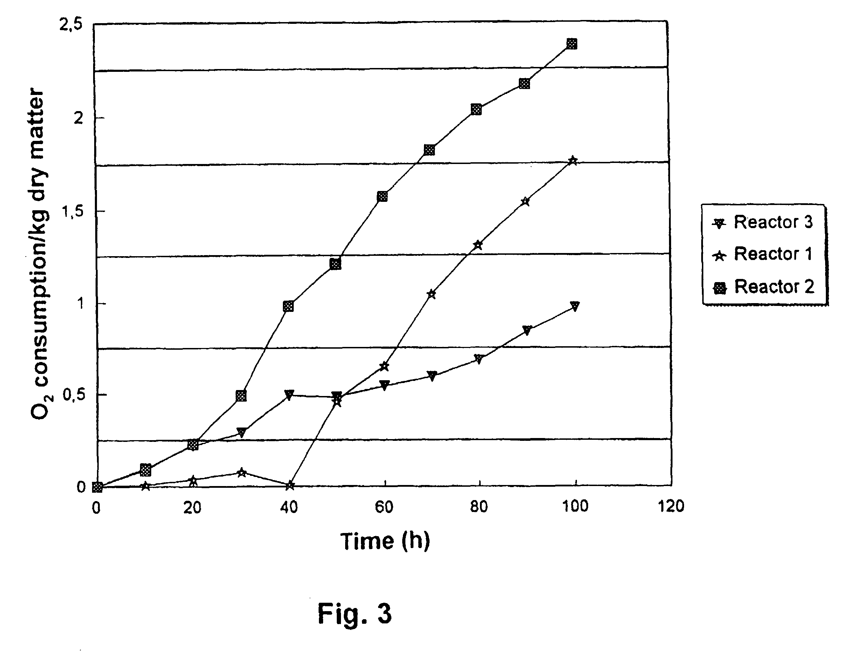

Reactor I contained biowaste having been treated with 1.5% potassium diformate and mixed with 1 weight% inoculate. Reactor II contained biowaste treated with the same amount of diformate and this was mixed with 20 weight% inoculate. Reactor III is the control reactor. The results of these experiments are shown in Figure 3.

The diformate was completely degraded and provided that not too much diformate had been used during the treatment of the waste prior to the composting process, it performed well. Accordingly, biowaste treated with diformate during its storage in collecting bins for up to three weeks, can be further processed by composting. In case of low microbial activity at the start of the composting process, this can easily be speeded up by addition of minor amounts inoculant as shown in Figure 3.

From the above examples it can be seen that the new way of treating biowaste results in substantial reduction of odour and microbial activity during collection and storage of the biowaste. The treatment agent is biodegradable, and the thus treated biowaste is useful for further processing.

## Claims

1. Method for treatment of househol biowaste collected in containers or bins, for reduction of odour and microbial activity, comprising addition of a treatment agent at least once during a storage period prior to further treatment of said biowaste, wherein said treatment agent is an aqueous solution of formate, acetate or propriotrate, comprising their respective acids and their disalts, which is sprayed on the biowaste,
**characterized in that**
said aqueous solution is sprayed in amounts of 1-3 weight%, calculated as formate/acetate/propionate equivalents, on the biowaste having a moisture content adjusted to 50-75 weight%.

2. Method according to claim 1,
**characterized in that**
an aqueous solution of sodium-, potassium- or ammonium diformate is applied.

3. Method according to claim 1,
**characterized in that**
a solution of 15-50 weight% potassium diformate is sprayed on the biowaste each time it is supplied to the bin in amounts of 50-70 ml/kg wet biowaste.

4. Method according to claim 1,
**characterized in that**
the aqueous solution applied comprises formic acid and formate, in a ratio of 1:1 to 1:2.

5. Method according to claim 1,
**characterized in that**
the treated biowaste is further processed to compost by addition of dry biowaste like bark, turf etc. in amounts of 25-75 weight% of the total mixture and that said mixture is aerated.

6. Method according to claim 5,
**characterized in that**
before or during the initial composting process inoculation of the mixture with adapted micro-organisms is performed.

7. Method according to claim 5,
**characterized in that**
the inoculation is performed by adding minor amounts of untreated biowaste or compost.

8. Use of sodium, ammonium or potassium mono- or disalts of formic-, acetic- or propionic acid, comprising their respective acids, for treatment of household biowaste having a moisture content adjusted to 50-75 weight%.

9. Use according to claim 8, of potassium diformate, comprising formic acid, for treatment of the biowaste.

## Patentansprüche

1. Verfahren zur Behandlung von Bioabfällen aus dem Haushalt, die in Containern oder Behältern gesammelt werden, zwecks Verminderung der Geruchsbelästigung und der mikrobiologischen Aktivität, wobei das Verfahren die Zugabe eines Behandlungsmittels umfasst, dies mindestens einmal während der Lagerperiode, bevor der Bioabfall einer weiteren Behandlung unterzogen wird, wobei das Behandlungsmittel eine wässrige Lösung von Formiaten, Acetaten oder Propionaten ist, die ihre jeweiligen Säuren und ihre Doppelsalze enthalten, und dieses Mittel auf den Bioabfall gesprüht wird,
**dadurch gekennzeichnet, dass**
die besagte wässrige Lösung in Mengen von 1-3 Gewichts%, berechnet als Formiat/Acetat/Propionat-Äquivalente, auf den Bioabfall aufgesprüht wird, der einen Feuchtigkeitsgehalt aufweist, der auf 50-75 Gewichts-% eingestellt ist.

2. Verfahren gemäss Anspruch 1,
**dadurch gekennzeichnet, dass**
eine wässrige Lösung von Natrium-, Kalium- oder Ammoniumdiformiat verwendet wird.

3. Verfahren gemäss Anspruch 1,
**dadurch gekennzeichnet, dass**
jedes Mal, wenn Bioabfall in den Sammelbehälter gefüllt wird, eine Lösung von 15-50 Gewichts-% Kaliumdiformiat auf den Bioabfall gesprüht wird, in Mengen von 50-70 ml/kg des feuchten Bioabfalls.

4. Verfahren gemäss Anspruch 1,
**dadurch gekennzeichnet, dass**
die eingesetzte wässrige Lösung die Ameisensäure und das Formiat in einem Verhältnis von 1:1 bis zu 1:2 enthält.

5. Verfahren gemäss Anspruch 1,
**dadurch gekennzeichnet, dass**
der behandelte Bioabfall weiter zu Kompost verarbeitet wird durch die Zugabe von trockenem Bioabfall, wie etwa Rinde, Torf usw. in Mengen von 25-75 Gewichts-%, bezogen auf die Gesamtmischung, und dass diese Mischung dann belüftet wird.

6. Verfahren gemäss Anspruch 5,
**dadurch gekennzeichnet, dass**
vor oder während des anfänglichen Prozesses der Kompostierung eine Einimpfung der Mischung mit angepassten Mikroorganismen durchgeführt wird.

7. Verfahren gemäss Anspruch 5,
**dadurch gekennzeichnet, dass**
die Einimpfung durchgeführt wird durch die Zugabe von kleineren Mengen an unbehandeltem Bioabfall oder an Kompost.

8. Verwendung von Einfach- oder Doppelsalzen von Ameisen-, Essig- oder Propionsäure mit Natrium, Ammonium oder Kalium, die ihre jeweiligen Säuren enthalten, für die Behandlung von Bioabfall aus dem Haushalt, der einen Feuchtigkeitsgehalt aufweist der auf 50-75 Gewichts-% angepasst ist.

9. Verwendung gemäss Anspruch 8 von Kaliumdiformiat, das Ameisensäure enthält, für die Behandlung von Bioabfall.

## Revendications

1. Procédé pour le traitement de déchets biologiques ménagers récupérés dans des conteneurs ou des bennes, pour la réduction de l'odeur et de l'activité microbienne, comprenant l'ajout d'un agent de traitement au moins une fois durant une période de stockage avant un autre traitement desdits déchets biologiques,
dans lequel ledit agent de traitement est une solution aqueuse de formate, d'acétate ou de propionate, comprenant leurs acides respectifs et leurs disels, qui est aspergée sur les déchets biologiques,
**caractérisé en ce que**
ladite solution aqueuse est aspergée dans des quantités de 1-3% en poids, calculées sous forme d'équivalents de formate/acétate/propionate, sur les déchets biologiques présentant une teneur en humidité ajustée à 50-75% en poids.

2. Procédé suivant la revendication 1,
**caractérisé en ce que**
une solution aqueuse de diformate de sodium, de potassium ou d'ammonium est appliquée.

3. Procédé suivant la revendication 1,
**caractérisé en ce que**
une solution de 15-50% en poids de diformate de potassium est aspergée sur les déchets biologiques chaque fois qu'ils sont introduits dans la benne dans des quantités de 50-70 ml/kg de déchets biologiques humides.

4. Procédé suivant la revendication 1,
**caractérisé en ce que**
la solution aqueuse appliquée comprend de l'acide formique et du formate, en un rapport de 1:1 à 1:2.

5. Procédé suivant la revendication 1,
**caractérisé en ce que**
les déchets biologiques traités sont en outre transformés en compost par l'ajout de déchets biologiques secs tels que des écorces,_du gazon, etc., dans des quantités de 25-75% en poids du mélange total et **en ce que** ledit mélange est aéré.

6. Procédé suivant la revendication 5,
**caractérisé en ce que**
avant ou pendant le procédé de compostage initial, une inoculation du mélange avec des micro-organismes adaptés est effectuée.

7. Procédé suivant la revendication 5,
**caractérisé en ce que**
l'inoculation est effectuée en ajoutant des quantités mineures de déchets biologiques non traités ou de compost.

8. Utilisation de mono- ou de disels de sodium, d'ammonium ou de potassium d'acide formique, acétique ou propionique, comprenant leurs acides respectifs, pour un traitement de déchets biologiques ménagers présentant une teneur en humidité ajustée à 50-75% en poids.

9. Utilisation suivant la revendication 8 de diformate de potassium, comprenant de l'acide formique, pour un traitement de déchets biologiques.
